# EUROPEAN PATENT SPECIFICATION

(11) **EP 1 381 438 B1**
(45) Date of publication and mention of the grant of the patent: **30.05.2012**
(21) Application number: 00988255.6
(22) Date of filing: 21.12.2000
(51) Int. Cl.: A61M 1/36, B01D 61/18, B01D 61/20, B01D 63/08

(54) **BIOLOGICAL FLUID FILTER**
FILTER FÜR BIOLOGISCHES FLUID
FILTRE DE FLUIDES BIOLOGIQUES

(43) Date of publication of application: 21.01.2004
(60) Divisional application: 10012142.5
(73) Proprietor: Hemerus Medical, LLC, St. Paul, MN 55112 (US)
(72) Inventor: ZIA, Majid, White Bear Township, MN 55127 (US); KHAMIS, Chaouki, A., Minneapolis, MN 55432 (US)
(74) Representative: Gleiter, Hermann
(86) International application number: PCT/US2000/034856
(87) International publication number: WO 2002/051520

(56) References cited:
- EP-A- 0 489 403
- WO-A-98/39080
- GB-A- 1 401 382
- US-A- 3 803 810
- US-A- 4 009 714
- US-A- 4 906 260
- US-A- 4 925 572

## Description

The present invention relates to a biological fluid filter for use in filtering biological fluid products into their therapeutically valuable components. The filtration media used in the biological fluid filters of the prior art are chosen or modified in order to have a critical wetting surface tension (CWST) in the proximity of the surface tension (ST) of the biological fluid to be filtered. If the CWST of the media is substantially lower that the ST of the biological fluid being filtered, the priming time for the filter increases as the difference between the CWST and the ST increases.

If the CWST of the media is substantially higher that the ST of the fluid, the filter media is easily wetted by the biological fluid. In cases where the filter media is easily wetted by the biological fluid, air may be entrapped within the filter device, which results is a reduction of the usable filter area, and decreased performance.

In US 4,906,260 a membrane-type filter device is described where vents located opposite the hydrophilic membrane remove entrapped gases in the inlet chamber of a housing. There can be two such vents on either end of the inlet chamber, the corners of which can be non-rectangular, which remove entrapped gases from the fluid without regard to filter device orientation.

In the present invention, a filter device according to claim 1 is introduced which alleviates or minimizes gas entrapment. The device is not limited by the CWST of the filter media, or the ST of the fluid to be filtered. Also, the device improves the filter priming time.

These benefits are achieved by dividing the filter medium into one or more sections, separated from each other, so that the wicking properties of the filter medium used will not cause any substantial air entrapment in the biological fluid filter device.

### BRIEF DESCRIPTION OF THE DRAWINGS

Fig. 1 is a perspective view of a biological fluid filter construction embodying the present invention;
Fig. 2 is a front elevational view of the construction shown in Fig. 1;
Fig. 3 is a sectional view, taken in the direction of the arrows, along the section line 3-3 of Fig. 2;
Fig. 4 is a sectional view, taken in the direction of the arrows, along the section line 4-4 of Fig. 2;
Fig. 5 is a sectional view, taken in the direction of the arrows, along the section line 5-5 of Fig. 2;
Fig. 6 is a front elevational view of the inlet portion of the construction shown in Fig. 1;
Fig. 7 is a rear elevational view of the construction shown in Fig. 6;
Fig. 8 is a front elevational view of the outlet portion of the construction shown in Fig. 1;
Fig. 9 is a rear elevational view of the construction shown in Fig. 8;
Fig. 10 is a modification of the construction shown in Fig. 1;
Fig. 11 is a diagrammatic view of the filter medium shown in the construction of Fig. 10;
Fig. 12 is a diagrammatic view of a further modification of the construction shown in Fig. 1; and
Fig. 13 is a diagrammatic view of the filter medium shown in the construction shown in Fig. 12.

It is to be understood that the present invention is not limited in its application to the details of construction and arrangement of parts illustrated in the accompanying drawings, since the invention is capable of other embodiments, and of being practiced or carried out in various ways within the scope of the claims. Also, it is to be understood that the phraseology and terminology employed herein is for the purpose of description, and not of limitation.

### DESCRIPTION OF THE PREFERRED EMBODIMENT

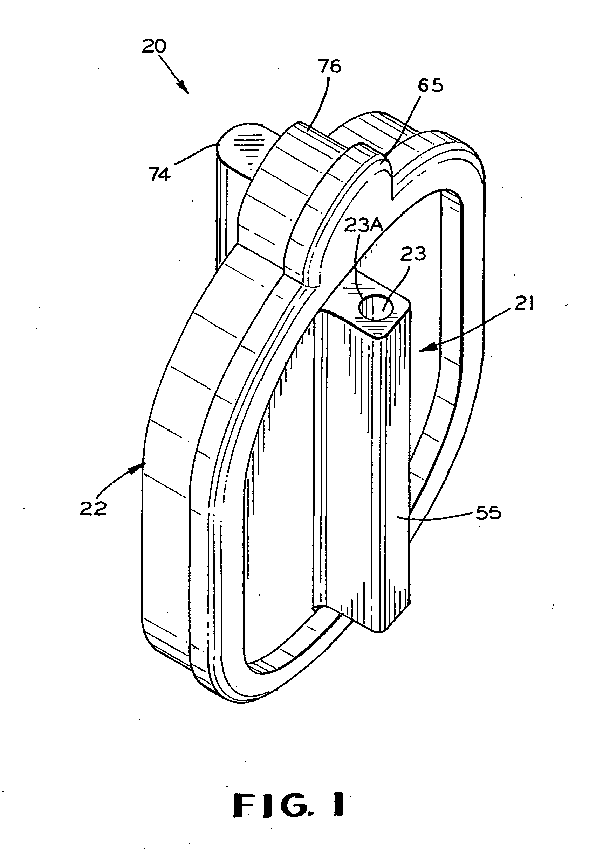

Referring to Fig. 1, there is shown a biological fluid filter, generally indicated by the numeral 20. The biological fluid filter device 20 may be such as is used in a system for processing a biological fluid, such as blood, into its therapeutically valuable components. The present invention is particularly useful for preventing gas entrapment in the upstream or inlet chamber of a biological fluid filter.

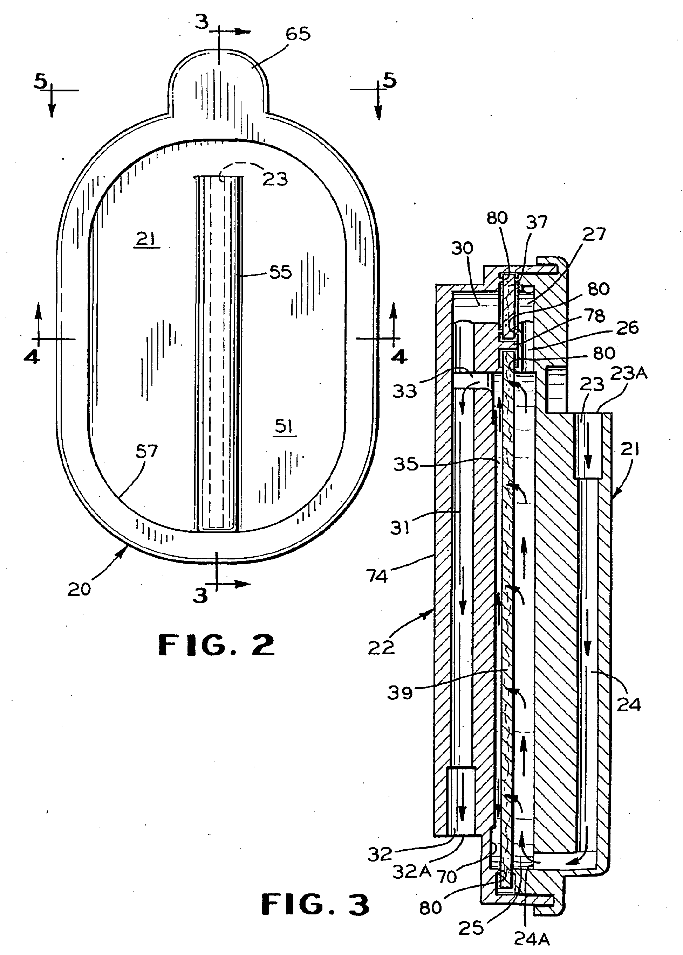

The biological fluid filter 20 consists of an inlet section 21 and an outlet section 22. Referring to Figs. 2 and 3, the inlet section 21 of biological fluid filter 20 has an inlet 23, including port 23A, communicating with first passage 24, which is in fluid communication with first or inlet chamber 25 through first port or outlet 24A. Further, biological fluid filter 20 has a second passage 26 in fluid communication with both, first or inlet chamber 25, and first vent chamber 27.

The outlet section 22 has a second vent chamber 30 in fluid communication with a third passage 31. Third passage 31 is in fluid communication with outlet 32 through port 32A. A fourth passage 33 is in communication with the third passage 31 and the second or outlet chamber 35. A vent filter element 37 separates the first vent camber 27 from the second vent chamber 30, and is held in place by means to be described in more detail hereinbelow.

Similarly, a biological filter element 39 separates the first or inlet chamber 25 from the second or outlet chamber 35. Both the vent filter element 37 and the biological filter element 39 may consist of one or more layers, and be made of a wide variety of filter materials. In the embodiment illustrated, they are liquiphilic.

In the preferred embodiment, the vent filter element 37, and the biological fluid filter element 39, are made of the same filter medium, which may be such as glass or nylon fibers. In use, a fluid container (not shown), such as a blood container is placed in fluid communication with inlet port 23A. Similarly, a biological fluid receiving bag (not shown) is placed in fluid communication, by means well known in the art, with outlet port 32A. Fluid flow is initiated and blood flows in the inlet port 23A, through the first passage 24 and through first outlet 24A into inlet chamber 25.

In operation, as the blood enters the inlet chamber 25, the blood may wick into the filter element 39. The blood may wick into the filter element 39 faster, or slower, than the blood level rises in the first or inlet chamber 25. The rate at which the blood wicks into the filter element 39 will depend on the properties of the filter medium being chosen, and the biological fluid being filtered. These properties include the pore size of the medium, the density of the biological fluid, the surface tension of the biological fluid, and the contact angle of the solid-liquid-gas interface.

While the blood level is rising in the inlet chamber 25, any air entrapped in chamber 25 is either passing through a portion of the filter element 39 which is not yet wetted, or is proceeding through second passage 26 and being vented out the vent filter element 37.

As the blood level continues to rise in inlet chamber 25, at some point, the biological filter element 39 will be sufficiently "wetted", and the biological fluid being filtered will "breakthrough" the filter element 39, and will start flowing into outlet chamber 35. The fluid breakthrough depends on the pore size of the material, the surface tension and the contact angle, as well as the pressure differential across the filter element 39.

Due to the pressure differential across the biological filter element 39, the biological fluid continues to flow up into second passage 26. If the pressure differential is sufficient, the biological fluid will contact the vent filter element 37, which is the preferred embodiment. If the vent filter element 37 is also made of a liquiphilic media, it will become "wetted out". However, by this time all the gas entrapped in inlet chamber 25 has either passed previously through biological filter element 39 or through vent filter element 37 and accomplished one of the objects of the invention.

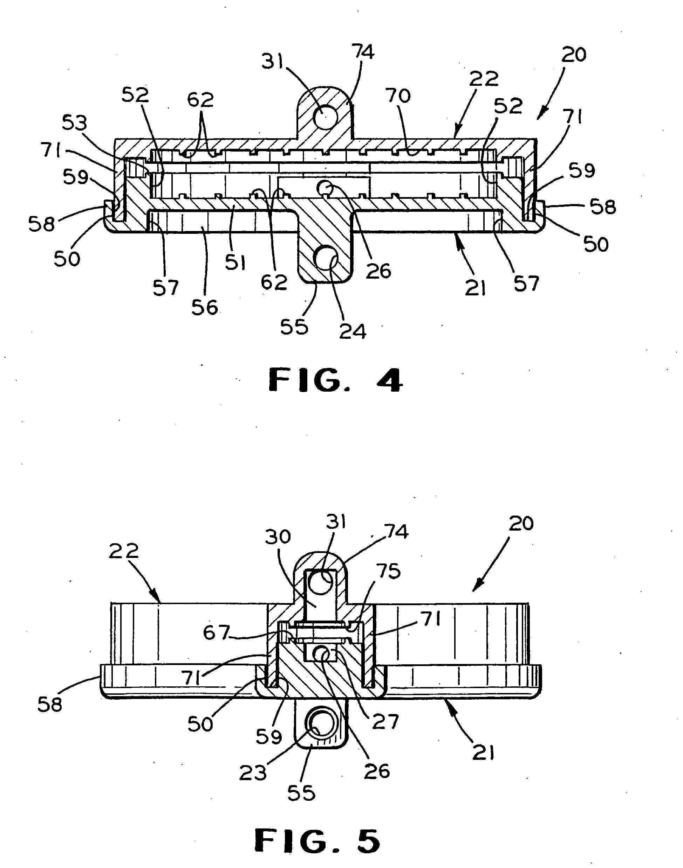

Referring now to Figs. 2-7, the construction of the inlet section 21 of the biological fluid filter 20 may be clearly understood. The biological fluid filter 20 includes an inlet section 21 which is bonded to an outlet section 22 by a seal 50. The seal 50 is preferably an ultrasonic seal. It can be understood by those skilled in the art that other seals such as heat seals, adhesive seals, or any other air tight seal may be used.

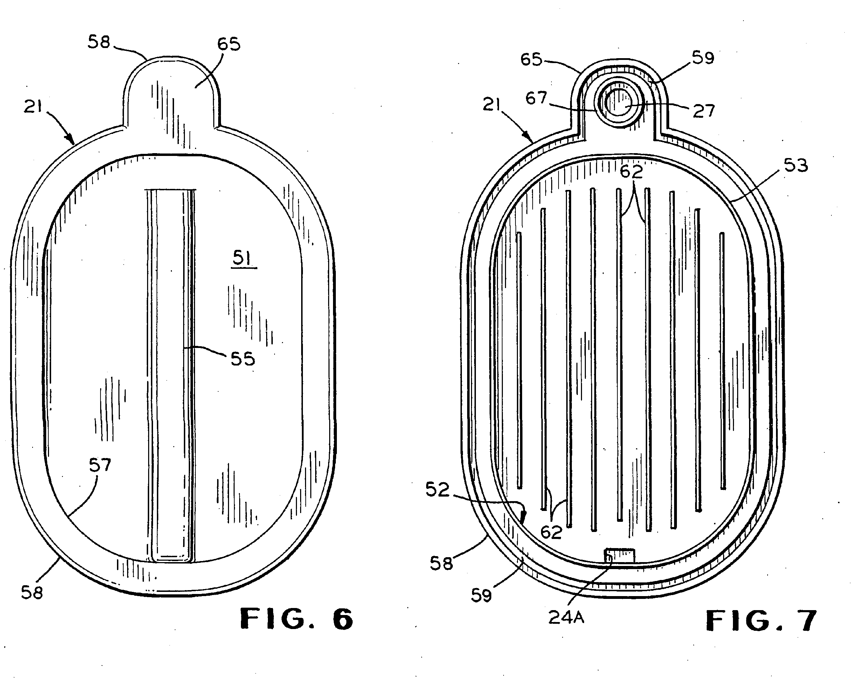

Inlet section 21 includes a recessed top wall 51, and downstanding side walls 52 extending around the periphery of the recessed top wall 51. A downstanding peripheral ridge 53 extends around the periphery of the downstanding side wall 52 and forms a part of the mechanism which holds the vent filter element 37 and the biological filter element 39 in place, as will be more fully explained hereinafter. A first protuberance 55 extends from the recessed top wall 51, and carries the inlet 23 and first passage 24 as previously described. First or outlet port 24A which is in fluid communication with the first passage 24 can be seen in Fig. 7. A recess 56, provided by the combination of the top surface of the recessed top wall 51 and the peripheral side walls 57 almost completely surrounds the protuberance 55.

A peripheral flange 58 depends from the peripheral sidewall 57 and forms a groove 59 extending around the periphery of the inlet section 21 of the biological fluid filter 20. The groove 59 forms a portion of the means by which the seal 50 between the inlet section 21 and the outlet section 22 of the biological fluid filter 20 is formed. A plurality of downstanding ribs 62 are provided on the lower surface of the recessed top wall 51 for purposes to be described.

The inlet portion 21 of the biological fluid filtration device also has an extended portion 65 which contains second passage 26 (Fig. 3) in fluid communication with first vent chamber 27. The same flange 58 and groove 59 are provided in the extended portion 65 of the inlet section 21 as are provided in the remainder of the inlet section 21, so that the inlet section 21 will properly mate with the outlet section 22 to be described. A circular ridge 67 is provided about the first vent chamber 27 to aid in holding the vent filter, as will be further described.

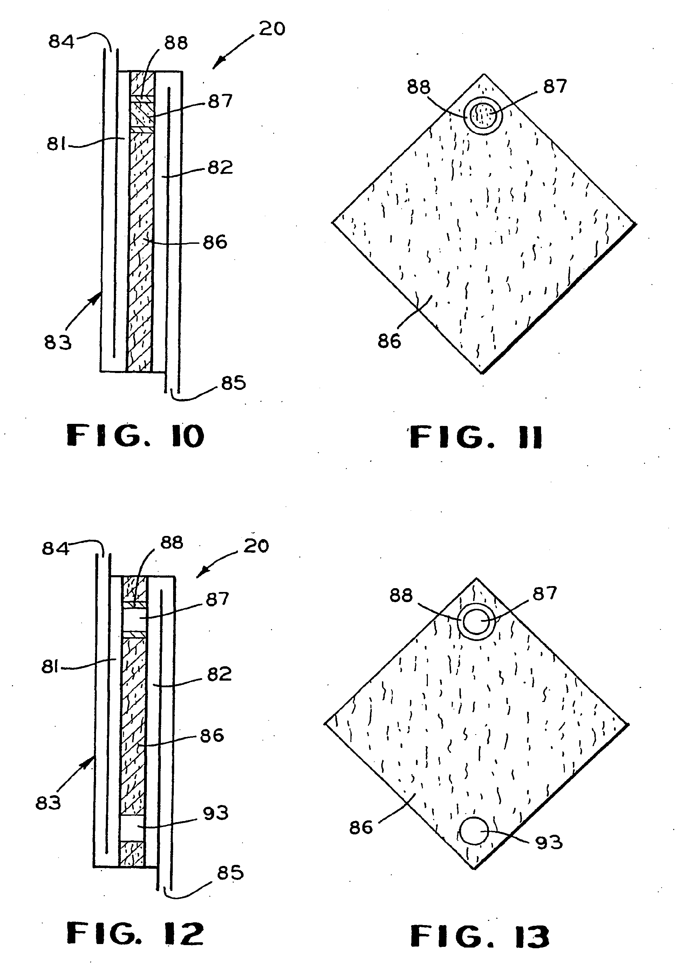

Referring now to Figs. 3-5 and 8-9, the construction of the outlet portion 22 of the biological fluid filter 20 will be clearly understood. The shape of the outlet section 22 of the biological fluid filter 20 is complimentary in shape to the inlet section 21 so that the inlet section 21 may act as a closure to the outlet section 22, or vice versa. It can easily be understood by those skilled in the art that the biological fluid filter 20 may be of any desired shape, such as the generally oval shape thus far described, the diamond shape of the modification shown in Figs. 10 or 12, or any other desired shape. Similar to the inlet section 21, the outlet section 22 of the biological fluid filter 20 has a bottom wall 70 and upstanding sidewall 71. The top of the upstanding sidewall 71 fits into the groove 59 in the inlet portion 21, and is preferably sonically welded to form the seal 50. A second protuberance 74 is provided on the exterior portion of the bottom wall 70 and carries the third passage 31, fourth passage 33, and a portion of the vent chamber 30. A second circular ridge 75, complimentary in shape to the circular ridge 67, is provided. The protuberance 74 covers a portion of the extended portion 76 of the outlet portion 22 of the biological fluid filter 20.

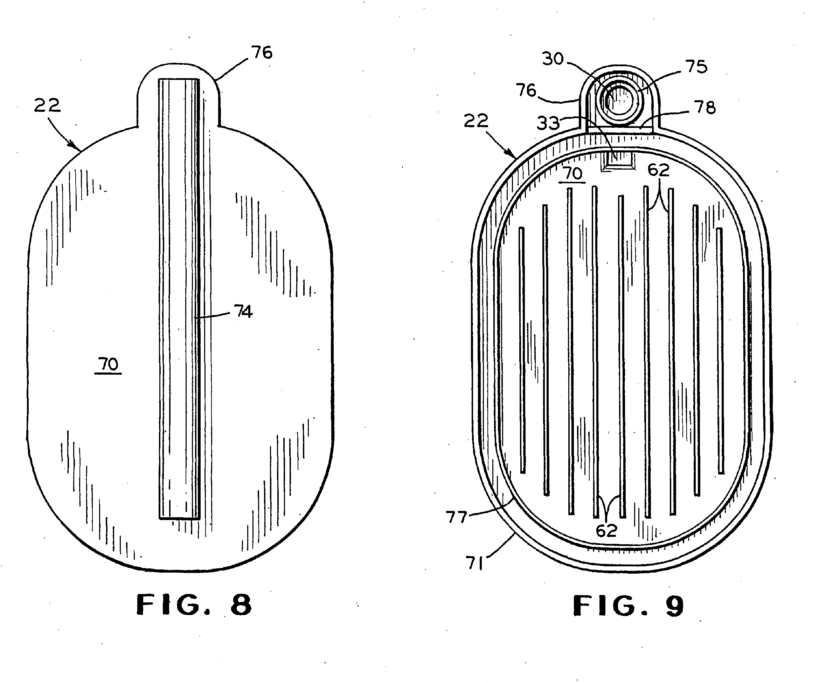

As seen in Fig. 9, a further plurality of ribs 62 is provided on the interior surface of the bottom wall 70 to help support the biological filter element 39 and provide flow in the second or outlet chamber 35 of the biological fluid filter 20. An upstanding ridge 77 is provided in a spaced apart relationship to the upstanding sidewall 71. As with the circular ridges 67 and 75, when the outlet portion 22 and the inlet portion 21 are in mating relationship, the downstanding ridge 53 and the upstanding ridge 77 will be in a 180° opposed relationship. As can be seen in Fig. 3 these ridges will provide the pinch seals 80 for the vent filter element 37 and the biological filter element 39. An ultrasonic weld ridge 78 is provided to separate vent filter 37 and biological filter element 39, and to provide additional support for the fluid filter 20.

Referring to Figs. 10 and 11, there is shown a modification of the biological fluid filter 20 previously described. In this modification of the invention, the biological fluid filter 20 has a housing 83, which may be constructed in a manner similar to that just described, or may be constructed by other means well known in the art. The housing has an inlet 84 to which a biological fluid container of the type well known in the art would be in fluid communication during operation. The housing 83 also has an outlet 85 through which the filtered fluid passes. The outlet 85 would be in fluid communication with a biological receiving container (not shown).

A filter element 86 would be sealingly mounted within the housing between inlet 84 and outlet 85. In this modification of the biological fluid filter 20, instead of there being a separate and distinct vent filter element 37, the vent filter element 37 is embedded in the biological fluid filter element 86. The biological filter element 86 may be made of a liquiphilic filter medium, and the embedded vent filter element 87 may also be made of a liquiphilic filter medium, surrounded by a solid or liquiphobic barrier 88. In operation, this modification of the biological fluid filter would operate in a similar manner to that just described because of the liquiphilic nature of the biological filter element 86, until the element was completely saturated. As the blood was rising in the inlet chamber 81, any entrapped gas would pass through the embedded vent filter element 87 until the level of the blood surpassed the solid or liquiphobic barrier 88. At this time, virtually all of the entrapped gas would be downstream of the biological filter element 86, the element would be completely saturated, and blood would now freely flow into the outlet chamber 82.

Another modification of the biological fluid filter 20 is shown in Figs. 12 and 13. As before, there is a filter housing 83 having an inlet 84 communicating with an inlet chamber 81, and an outlet 85 communicating with an outlet chamber 82. In this modification of the invention, the biological filter element 86 has a first embedded liquiphilic gas vent 87 surrounded by a solid barrier 88, and a second embedded liquiphobic gas vent 93.

In operation, a biological fluid container known in the art (not shown) will be in fluid communication with inlet 84. As blood is released from the biological fluid container it will flow into the inlet chamber 81 and come into contact with the bottom of the biological fluid filter element 86. Since filter element 86 may be a liquiphilic porous medium, the blood level may wick up in the liquiphilic porous medium 86 faster than the level in the chamber 81. The blood will not pass through the liquiphobic second embedded gas vent 93. The second embedded gas vent 93 will have no effect on the operation of the biological fluid filter 20 while the liquid level continues to rise in inlet chamber 81. However, the difference between the embodiment of the invention shown in Figs. 10 and 11, and 12 and 13, becomes apparent when all of the blood has been released from the biological filter container and the level starts dropping in the inlet chamber 81. The vent filter element 87 will stay wetted out as the level in the inlet chamber 81 drops because of the blood present in the outlet chamber 82. However, as the level in the inlet chamber 81 continues to drop it will drop below the level of the liquiphobic second embedded gas vent 93. Since gas vent 93 did not wet out, when the blood level drops, air will pass from the inlet chamber 81 through the second embedded gas vent 93, and aid in draining the filter element 86, as well as the outlet chamber 82, through the outlet 85.

Thus, a new and novel biological fluid filtration device has been developed which can use a wide range of filter media, and filter a wide range of biological fluids, without being limited by the CWST of the media, or the ST of the biological fluid being filtered.

## Claims

1. A biological fluid filter comprising
an inlet section (21), said inlet section comprising a top wall (51) and a downstanding peripheral side wall (52),
wherein said inlet section further comprises an inlet (23), a first passage (24) in fluid communication with said inlet, and a first outlet in fluid communication witch said first passage and an inlet chamber (25),
and wherein the biological fluid filter further comprises
an outlet section (22) complimentary in shape to and bonded to said inlet section to form a chamber communicating with atmosphere, said outlet section comprising a bottom wall (70) and an upstanding peripheral side wall (71), and
a filter element (39) mechanically held in place between said inlet section and said outlet section to divide said chamber into at least said inlet chamber (25) and an outlet chamber (35),
**characterized in that**
the biological fluid filter has a second passage (26) in fluid communication with both the inlet chamber and a first vent chamber (27),
the outlet section comprises a second vent chamber (30) in fluid communication with a third passage (31), and a fourth passage (33) which is in communication with the third passage and the outlet chamber, and
further **characterized by**
a vent filter element (37) separating the first vent chamber from the second vent chamber and being mechanically held in place between said first vent chamber and said second vent chamber.

2. The biological fluid filter according to claim 1, wherein
the inlet section has the inlet (23), the first passage (24) in fluid communication with said inlet, a first port (23A) in fluid communication with said first passage (24), the inlet chamber (25) in fluid communication with said first port, and the first vent chamber (27); and
the outlet section (22) comprises the outlet chamber (35), the fourth passage (33) in fluid communication with said outlet chamber (35), the third passage (31) in fluid communication with said fourth passage, and an outlet (32) in fluid communication with said fourth passage (33);
and the filter element (39) separating said inlet chamber from said outlet chamber, wherein the filter element is a biological filter element.

3. The biological fluid filter of claim 2, wherein said inlet section has said inlet and wherein said first port is an inlet port for communication with said first passage.

4. The biological fluid filter of claim 2, wherein said third passage is in fluid communication with the outlet through an outlet port.

5. The biological fluid filter of claim 2, wherein said vent filter element and said biological filter element consist of at least one layer.

6. The biological fluid filter of claim 2, wherein said vent filter element and said biological filter element are liquiphilic.

7. The biological fluid filter of claim 2, wherein said vent filter element and said biological filter element are made of the same filter medium.

8. The biological fluid filter of claim 2, wherein said inlet section is bonded to said outlet section by an air tight seal.

9. The biological fluid filter of claim 8, wherein said seal is an ultrasonic seal.

10. The biological fluid filter of claim 2, wherein said inlet section includes a recessed top wall and a downstanding sidewall peripherally extending from said top wall.

11. The biological fluid filter of claim 10, wherein a downstanding peripheral ridge extends around the periphery of said downstanding sidewall.

12. The biological fluid filter of claim 10, wherein a first protuberance extends from said recessed top wall, carrying said inlet and said first passage.

13. The biological fluid filter of claim 10, wherein a peripheral flange depends from said peripheral sidewall and forms a groove extending around the periphery of said inlet section.

14. The biological fluid filter of claim 13, wherein said groove forms a portion of the means by which a seal between said inlet section and said outlet section is formed.

15. The biological fluid filter of claim 10, wherein said recessed top wall is provided with a lower surface.

16. The biological fluid filter of claim 15, wherein said lower surface is provided with a plurality of downstanding ribs.

17. The biological fluid filter of claim 2, wherein said inlet section has an extended portion containing a second passage in fluid communication with said first vent chamber.

18. The biological fluid filter of claim 17, wherein said extended portion is provided with flange and groove for proper mating with said outlet section.

19. The biological fluid filter of claim 2, wherein said first vent chamber is provided with a circular ridge to aid in holding said vent filter.

20. The biological fluid filter of claim 2, wherein said outlet section is complimentary in shape to said inlet section allowing said inlet section to act as a closure to said outlet section, or vice versa.

21. The biological fluid filter of claim 2, wherein said upstanding sidewall fits into said groove in said inlet section.

22. The biological fluid filter of claim 21, wherein said upstanding sidewall is sonically welded to form a seal with said inlet section.

23. The biological fluid filter of claim 22, wherein an upstanding ridge is provided in a space apart relationship to said upstanding sidewall.

24. The biological fluid filter of claim 23, wherein when said outlet portion and said inlet portion are in a mating relationship, said upstanding ridge and said downstanding ridge form pinch seals for said vent filter element and said biological filter element.

25. The biological fluid filter of claim 2, wherein said bottom wall has an exterior and an interior.

26. The biological fluid filter of claim 25, wherein said exterior of said bottom wall is provided with a second protuberance.

27. The biological fluid filter of claim 26, wherein said second protuberance carries said third passage, said fourth passage, and a portion of said second vent chamber.

28. The biological fluid filter of claim 26, wherein said second protuberance covers a portion of an extended portion of said outlet portion.

29. The biological fluid filter of claim 25, wherein a plurality of ribs is provided on said interior surface of said bottom wall to help support said biological filter element and provide flow in said second chamber.

30. The biological fluid filter of claim 2, wherein an ultrasonic weld ridge is provided to separate said vent filter and said biological filter element and to provide additional support for said filter element.

## Patentansprüche

1. Biologischer Fluidfilter, aufweisend
einen Einlassbereich (21), wobei besagter Einlassbereich eine obere Wand (51) und eine herunterstehende senkrechte Seitenwand (52) aufweist,
wobei besagter Einlassbereich außerdem einen Einlass (23), einen ersten Durchfluss (24) in Fluidkommunikation mit besagtem Einlass, und einen ersten Auslass in Fluidkommunikation mit dem besagten ersten Durchfluss und eine Einlasskammer (25) aufweist,
und wobei der biologische Fluidfilter weiter aufweist einen Auslassbereich (22) komplementär in Form zu und verbunden mit besagtem Einlassbereich, um eine Kammer zu bilden, die mit der Atmosphäre kommuniziert, wobei besagter Auslassbereich eine untere Wand (70) und eine hochstehende periphere Seitenwand (71) aufweist, und
ein Filterelement (39), das mechanisch zwischen besagtem Einlassbereich und besagtem Auslassbereich am Ort gehalten wird, um besagte Kammer in zumindest besagte Einlasskammer (25) und eine Auslasskammer (35) zu unterteilen,
**dadurch gekennzeichnet, dass**
der biologische Fluidfilter einen zweiten Durchfluss (26) in Fluidkommunikation mit sowohl der Einlasskammer als auch einer ersten Belüftungskammer (27) hat,
der Auslassbereich eine zweite Belüftungskammer (30) in Fluidkommunikation mit einem dritten Durchfluss (31) aufweist, einen vierten Durchfluss (33), der in Kommunikation mit dem dritten Durchfluss und der Auslasskammer ist, und
weiter **gekennzeichnet durch**
ein Belüftungsfilterelement (37), das die erste Belüftungskammer von der zweiten Belüftungskammer trennt und mechanisch zwischen der besagten ersten Belüftungskammer und der besagten zweiten Belüftungskammer am Ort gehalten wird.

2. Biologischer Fluidfilter nach Anspruch 1, wobei der Einlassbereich den Einlass (23), den ersten Durchfluss (24) in Fluidkommunikation mit besagtem Einlass, einen ersten Anschluss (23A) in Fluidkommunikation mit besagtem ersten Durchfluss (24) hat, wobei die Einlasskammer (25) in Fluidkommunikation mit dem besagten ersten Anschluss und der ersten Belüftungskammer (27) ist; und
der Auslassbereich (22) die Auslasskammer (35), den vierten Durchfluss (33) in Fluidkommunikation mit besagter Auslasskammer (35), den dritten Durchfluss (31) in Fluidkommunikation mit dem besagten vierten Durchfluss, und ein Auslass (32) in Fluidkommunikation mit besagtem vierten Durchfluss (33) aufweist;
und wobei das Filterelement (39) die besagte Einlasskammer von der besagten Auslasskammer trennt, wobei das Filterelement ein biologisches Filterelement ist.

3. Biologischer Fluidfilter nach Anspruch 2, wobei der besagte Einlassbereich besagten Einlass aufweist und wobei der besagte erste Anschluss ein Einlassanschluss zur Kommunikation mit besagtem ersten Durchfluss ist.

4. Biologischer Fluidfilter nach Anspruch 2, wobei der dritte Durchfluss in Fluidkommunikation mit dem Auslass durch einen Auslassanschluss ist.

5. Biologischer Fluidfilter nach Anspruch 2, wobei das besagte Belüftungsfilterelement und das besagte biologische Filterelement aus zumindest einer Schicht bestehen.

6. Biologischer Fluidfilter nach Anspruch 2, wobei das besagte Belüftungsfilterelement und das besagte biologische Filterelement liquiphil sind.

7. Biologischer Fluidfilter nach Anspruch 2, wobei das besagte Belüftungsfilterelement und das besagte biologische Filterelement aus dem gleichen Filtermedium gemacht sind.

8. Biologischer Fluidfilter nach Anspruch 2, wobei der besagte Einlassbereich mit dem besagten Auslassbereich über eine luftdichte Dichtung verbunden ist.

9. Biologischer Fluidfilter nach Anspruch 8, wobei die besagte Dichtung eine Ultraschalldichtung ist.

10. Biologischer Fluidfilter nach Anspruch 2, wobei der besagte Einlassbereich eine zurückgesetzte obere Wand und eine herunterstehende Seitenwand, die sich peripher von besagter oberer Wand erstreckt, enthält.

11. Biologischer Fluidfilter nach Anspruch 10, wobei sich ein herunterstehender peripherer Grat um die Peripherie der besagten herunterstehenden Seitenwand erstreckt.

12. Biologischer Fluidfilter nach Anspruch 10, wobei sich eine erste Protuberanz von besagter zurückgenommener oberer Wand erstreckt, die besagten Einlass und besagten ersten Durchfluss trägt.

13. Biologischer Fluidfilter nach Anspruch 10, wobei ein peripherer Flansch von besagter peripherer Seitenwand abhängt und eine Nut bildet, die sich um die Peripherie des besagten Einlassbereiches erstreckt.

14. Biologischer Fluidfilter nach Anspruch 13, wobei die besagte Nut einen Teil jener Mittel bildet, durch welche die Dichtung zwischen dem besagten Einlassbereich und dem besagten Auslassbereich gebildet wird.

15. Biologischer Fluidfilter nach Anspruch 10, wobei die besagte zurückgesetzte obere Wand mit einer unteren Oberfläche versehen ist.

16. Biologischer Fluidfilter nach Anspruch 15, wobei die besagte untere Oberfläche mit einer Vielzahl von herunterstehenden Rippen versehen ist.

17. Biologischer Fluidfilter nach Anspruch 2, wobei der besagte Einlassbereich einen erweiterten Bereich aufweist, der einen ersten Durchfluss in Fluidkommunikation mit der besagten ersten Belüftungskammer enthält.

18. Biologischer Fluidfilter nach Anspruch 17, wobei der erweiterte Bereich mit einem Flansch und einer Nut zum exakten Verbinden mit besagtem Auslassbereich versehen ist.

19. Biologischer Fluidfilter nach Anspruch 2, wobei die besagte erste Belüftungskammer mit einem kreisförmigen Grat zum Unterstützen des Haltens des besagten Belüftungsfilters versehen ist.

20. Biologischer Fluidfilter nach Anspruch 2, wobei der besagte Auslassbereich in seiner Form komplementär zu besagtem Einlassbereich ist und es ermöglicht, dass besagter Einlassbereich als ein Verschluss des besagten Auslassbereichs dient oder andersherum.

21. Biologischer Fluidfilter nach Anspruch 2, wobei die besagte hochstehende Seitenwand in besagte Nut in besagtem Einlassbereich passt.

22. Biologischer Fluidfilter nach Anspruch 21, wobei die besagte hochstehende Seitenwand ultraschallgeschweißt ist, um eine Dichtung mit besagtem Einlassbereich zu bilden.

23. Biologischer Fluidfilter nach Anspruch 22, wobei ein hochstehender Grat in einer Beabstandungsbeziehung zu besagter hochstehender Seitenwand vorgesehen ist.

24. Biologischer Fluidfilter nach Anspruch 23, wobei der besagte Auslassbereich und der besagte Einlassbereich in einer zusammen passenden Beziehung sind, wobei besagter hochstehender Grat und besagter herunterstehender Grat Quetschdichtungen für das besagte Belüftungsfilterelement und das besagte biologische Filterelement bilden.

25. Biologischer Fluidfilter nach Anspruch 2, wobei die besagte untere Wand ein Äußeres und ein Inneres aufweist.

26. Biologischer Fluidfilter nach Anspruch 25, wobei das besagte Äußere der besagten unteren Wand mit einer zweiten Protuberanz versehen ist.

27. Biologischer Fluidfilter nach Anspruch 26, wobei die besagte zweite Protuberanz den besagten dritten Durchfluss, den besagten vierten Durchfluss und einen Teil der besagten zweiten Belüftungskammer trägt.

28. Biologischer Fluidfilter nach Anspruch 26, wobei die besagte zweite Protuberanz einen Teil eines erweiterten Teils des besagten Auslassbereichs bedeckt.

29. Biologischer Fluidfilter nach Anspruch 25, wobei eine Vielzahl von Rippen auf besagter innerer Oberfläche der besagten unteren Wand vorgesehen ist, um das Tragen des besagten biologischen Filterelementes zu unterstützen und Fluss in besagter zweiten Kammer bereitzustellen.

30. Biologischer Fluidfilter nach Anspruch 2, wobei ein Ultraschallschweißgrat vorgesehen ist, um den besagten Lüftungsfilter und das besagte biologische Filterelement zu trennen und zusätzliche Unterstützung für das besagte Filterelement zur Verfügung zu stellen.

## Revendications

1. Filtre de fluides biologiques comprenant :
une section d'entrée (21), ladite section d'entrée comprenant une paroi supérieure (51) et une paroi latérale périphérique descendante (52),
dans lequel ladite section d'entrée comprend en outre une entrée (23), un premier passage (24) en communication de fluide avec ladite entrée, et une première sortie en communication de fluide avec ledit premier passage et une chambre d'entrée (25),
et dans lequel le filtre de fluides biologiques comprend en outre :
une section de sortie (22) complémentaire du point de vue de la forme et reliée à ladite section d'entrée afin de former une chambre communiquant avec l'atmosphère, ladite section de sortie comprenant une paroi inférieure (70) et une paroi latérale périphérique ascendante (71), et
un élément de filtre (39) mécaniquement maintenu en place entre ladite section d'entrée et ladite section de sortie pour diviser ladite chambre en au moins ladite chambre d'entrée (25) et une chambre de sortie (35),
**caractérisé en ce que** :
le filtre de fluides biologiques a un deuxième passage (26) en communication de fluide, à la fois avec la chambre d'entrée et une première chambre de purge d'air (27),
la section de sortie comprend une deuxième chambre de purge d'air (30) en communication de fluide avec un troisième passage (31) et un quatrième passage (33), qui est en communication avec le troisième passage et la chambre de sortie, et
**caractérisé en outre par** :
un élément de filtre de purge d'air (37) séparant la première chambre de purge d'air de la deuxième chambre de purge d'air et étant mécaniquement maintenu en place entre ladite première chambre de purge d'air et ladite deuxième chambre de purge d'air.

2. Filtre de fluides biologiques selon la revendication 1, dans lequel :
la section d'entrée a l'entrée (23), le premier passage (24) en communication de fluide avec ladite entrée, un premier orifice (23A) en communication de fluide avec ledit premier passage (24), la chambre d'entrée (25) en communication de fluide avec ledit premier orifice et la première chambre de purge d'air (27) ; et
la section de sortie (22) comprend la chambre de sortie (35), le quatrième passage (33) en communication de fluide avec ladite chambre de sortie (35), le troisième passage (31) en communication de fluide avec ledit quatrième passage et une sortie (32) en communication de fluide avec ledit quatrième passage (33) ;
et l'élément de filtre (39) séparant ladite chambre d'entrée de ladite chambre de sortie, dans lequel l'élément de filtre est un élément de filtre biologique.

3. Filtre de fluides biologiques selon la revendication 2, dans lequel ladite section d'entrée a ladite entrée, et dans lequel ledit premier orifice est un orifice d'entrée pour la communication avec ledit premier passage.

4. Filtre de fluides biologiques selon la revendication 2, dans lequel ledit troisième passage est en communication de fluide avec la sortie, en passant par un orifice de sortie.

5. Filtre de fluides biologiques selon la revendication 2, dans lequel ledit élément de filtre de purge d'air et ledit élément de filtre biologique se composent d'au moins une couche.

6. Filtre de fluides biologiques selon la revendication 2, dans lequel ledit élément de filtre de purge d'air et ledit élément de filtre biologique sont liquophiles.

7. Filtre de fluides biologiques selon la revendication 2, dans lequel ledit élément de filtre de purge d'air et ledit élément de filtre biologique sont réalisés avec la même matière filtrante.

8. Filtre de fluides biologiques selon la revendication 2, dans lequel ladite section d'entrée est reliée à ladite section de sortie par un joint d'étanchéité à l'air.

9. Filtre de fluides biologiques selon la revendication 8, dans lequel ledit joint d'étanchéité est un joint à ultrasons.

10. Filtre de fluides biologiques selon la revendication 2, dans lequel ladite section d'entrée comprend une paroi supérieure évidée et une paroi latérale descendante s'étendant de manière périphérique à partir de ladite paroi supérieure.

11. Filtre de fluides biologiques selon la revendication 10, dans lequel une crête périphérique descendante s'étend autour de la périphérie de ladite paroi latérale descendante.

12. Filtre de fluides biologiques selon la revendication 10, dans lequel une première protubérance s'étend à partir de ladite paroi supérieure évidée, supportant ladite entrée et ledit premier passage.

13. Filtre de fluides biologiques selon la revendication 10, dans lequel un rebord périphérique dépend de ladite paroi latérale périphérique et forme une rainure s'étendant autour de la périphérie de ladite section d'entrée.

14. Filtre de fluides biologiques selon la revendication 13, dans lequel ladite rainure forme une partie des moyens grâce auxquels un joint d'étanchéité est formé entre ladite section d'entée et ladite section de sortie.

15. Filtre de fluides biologiques selon la revendication 10, dans lequel ladite paroi supérieure évidée est prévue avec une surface inférieure.

16. Filtre de fluides biologiques selon la revendication 15, dans lequel ladite surface inférieure est prévue avec une pluralité de nervures descendantes.

17. Filtre de fluides biologiques selon la revendication 2, dans lequel ladite section d'entrée a une partie étendue contenant un deuxième passage en communication de fluide avec ladite première chambre de purge d'air.

18. Filtre de fluides biologiques selon la revendication 17, dans lequel ladite partie étendue est prévue avec un rebord et une rainure pour se coupler correctement avec ladite section de sortie.

19. Filtre de fluides biologiques selon la revendication 2, dans lequel ladite première chambre de purge d'air est prévue avec une crête circulaire pour aider à maintenir ledit filtre de purge d'air.

20. Filtre de fluides biologiques selon la revendication 2, dans lequel ladite section de sortie est complémentaire du point de vue de la forme à ladite section d'entrée, permettant à ladite section d'entrée de servir de fermeture à ladite section de sortie ou vice-versa.

21. Filtre de fluides biologiques selon la revendication 2, dans lequel ladite paroi latérale ascendante s'adapte dans ladite rainure dans ladite section d'entrée.

22. Filtre de fluides biologiques selon la revendication 21, dans lequel ladite paroi latérale ascendante est soudée par ultrasons afin de former un joint d'étanchéité avec ladite section d'entrée.

23. Filtre de fluides biologiques selon la revendication 22, dans lequel une crête ascendante est prévue selon une relation espacée par rapport à ladite paroi latérale ascendante.

24. Filtre de fluides biologiques selon la revendication 23, dans lequel, lorsque ladite partie de sortie et ladite partie d'entrée sont en relation de couplage, ladite crête ascendante et ladite crête descendante forment des joints par pincement pour ledit élément de filtre de purge d'air et ledit élément de filtre biologique.

25. Filtre de fluides biologiques selon la revendication 2, dans lequel ladite paroi inférieure a un extérieur et un intérieur.

26. Filtre de fluides biologiques selon la revendication 25, dans lequel ledit extérieur de ladite paroi inférieure est prévu avec une deuxième protubérance.

27. Filtre de fluides biologiques selon la revendication 26, dans lequel ladite deuxième protubérance supporte ledit troisième passage, ledit quatrième passage et une partie de ladite deuxième chambre de purge d'air.

28. Filtre de fluides biologiques selon la revendication 26, dans lequel ladite deuxième protubérance recouvre une partie d'une partie étendue de ladite partie de sortie.

29. Filtre de fluides biologiques selon la revendication 25, dans lequel on prévoit une pluralité de nervures sur ladite surface intérieure de ladite paroi inférieure pour aider à supporter ledit élément de filtre biologique et fournir l'écoulement dans ladite deuxième chambre.

30. Filtre de fluides biologiques selon la revendication 2, dans lequel une crête soudée par ultrasons est prévue pour séparer ledit filtre de purge d'air et ledit élément de filtre biologique et pour fournir un support supplémentaire pour ledit élément de filtre.
